# EUROPEAN PATENT SPECIFICATION

(11) **EP 3 430 900 B1**
(45) Date of publication and mention of the grant of the patent: **04.03.2020**
(21) Application number: 17713334.5
(22) Date of filing: 09.03.2017
(51) Int. Cl.: A01M 1/22, A01M 1/06, A01M 1/02

(54) **DEVICE AND METHOD FOR THE SELECTIVE ELIMINATION OF PUPAE**
VORRICHTUNG UND VERFAHREN ZUR SELEKTIVEN BESEITIGUNG VON VERPUPPTEN MÜCKENLARVEN
DISPOSITIF ET PROCÉDÉ POUR L'ÉLIMINATION SÉLECTIVE DES CHRYSALIDES

(30) Priority: 14.03.2016 ES 201630293
(43) Date of publication of application: 23.01.2019
(73) Proprietor: Empresa De Transformación Agraria, S.A., 28006 Madrid (ES)
(72) Inventor: SALVADOR HERRANZ, Gustavo Manuel, 46182 La Cañada (Valencia) (ES); TUR LAHIGUERAM, Carles, 46950 Xirivella (Valencia) (ES); PLÁ MORA, Ignacio, 46701 Gandía (Valencia) (ES); DESCALZO DESCALZO, José Félix, 46315 Caudete de las Fuentes (Valencia) (ES)
(74) Representative: Covadonga Fernández-Vega Feijoo, Maria
(86) International application number: PCT/ES2017/070134
(87) International publication number: WO 2017/158216

(56) References cited:
- WO-A1-2012/171445
- WO-A1-2013/140167
- WO-A1-2015/160958
- GB-A- 1 295 942
- US-A1- 2002 121 045
- US-A1- 2007 074 447
- US-A1- 2007 214 711
- DA FOCKS: "An improved separator for the developmental stages, sexes, and species of mosquitoes", JOURNAL OF MEDICAL ENTOMOLOGY, vol. 17, no. 6, 30 December 1980 (1980-12-30), pages 567-568, XP055380390, cited in the application

## Description

### TECHNICAL FIELD

The present invention relates to a device and method for selectively eliminating mosquito pupae or the like, selecting the pupae by sex and rendering the unwanted pupae useless or killing them. The present invention can be applied in pupae breeding in an aquatic environment and having sexual dimorphism.

The present invention is applicable in the field of pest control and in caring for and breeding insects for laboratories, for example.

### PRIOR ART

For pest treatments or for treating other insect-related problems, a possibility of acting without insecticides and without any effects on other fauna is to sterilize the individuals of one sex and release them into nature so that they produce non-viable eggs, reducing the reproductive capacity of the species.

This case involves selecting the males, sterilizing them and releasing them into the environment to compete with wild males for the females. To that end, the males must be selected using any property that allows distinguishing them from the females.

The publication "An improved separator for the developmental stages, sexes, and species of mosquitoes" (Journal of Medical Entomology; Vol. 17, no. 6; pages 567-568; 30 December 1980) discloses a mechanical method for selecting pupae with sexual dimorphism, consisting of an adjustable through groove which allows smaller pupae to go through and retains larger pupae. This solution allows performing separation but requires intensive labor, and furthermore a considerable amount of unwanted individuals may be obtained after separating the individuals by sex.

### DISCLOSURE OF THE INVENTION

The invention consists of a device and method for selectively eliminating pupae according to the claims.

The developed system allows sexing mosquitoes and other insects or selecting them by sex taking into consideration the sexual dimorphism present between males and females when they are in the pupal stage. In this stage, males have a significantly smaller size than females do.

By means of using high-resolution cameras and artificial vision techniques, the system is capable of identifying the individuals separately, obtaining their size measured in square pixels. This feature allows establishing a size threshold differentiating males and females with certain precision.

Once the threshold is known, action (elimination from the flow) can be performed both on individuals exceeding said threshold and on smaller individuals, destroying them, for example, by means of a high-precision high-frequency laser that directly kills said individuals or prevents their evolution.

The device for selectively eliminating aquatic pupae of insects having sexual dimorphism of the invention comprises:
A feeder for feeding pupae in an aquatic environment on a conveyance system. This conveyance system is formed by a movable mesh moving at a constant and uniform speed, and a base. The mesh allows water to flow out, so the pupae are immobilized on the mesh in a horizontal position. The recognition and measurement thereof by means of artificial vision are therefore easier.
A lighting system, preferably backlighting system by means of DC LEDs.
One or more cameras connected to an artificial vision system locating the pupae and measuring their size. A size threshold or reference value distinguishing females from males will be defined with the measurements of each batch.
One or more eliminators for eliminating pupae from the flow, which eliminators will generally correspond to lasers that are preferably directable by means of respective redirecting galvanometers. Since the pupae are arranged in dry form on the mesh, there are no power losses and it is easier to aim before firing. Other mechanical methods for eliminating pupae from the flow (with or without destroying the pupa) would be to perform suction or blowing on a reject container, crushing, cutting, ultrasounds, etc. The laser and suction are preferred methods due to their precision in the case of elimination with or without destroying pupae.
A control system selecting pupae having a size larger or smaller than the threshold for being eliminated.
A removal system for removing the pupae from the conveyance system.

The cameras can be divided into two groups: a sampling camera in a sampling area before a positioning camera the field of vision of which coincides with the area in which the eliminators are effective, for example a laser firing area.

The conveyance system can be a rotary or conveyor belt conveyance system.

The method of the invention in turn comprises the steps of:
a) Feeding the pupae in an aquatic environment to a conveyance system with a movable mesh.
b) Taking an image of all the pupae by means of at least one camera.
c) Sorting the size of the pupae by means of artificial vision and defining a separation threshold between males and females. The females will have a larger size in most species. The threshold can be defined in a dynamic manner, such that it changes continuously according to the effective size of the introduced pupae.
d) Eliminating (destroying or removing from the flow) selected pupae above or below the threshold by means of a laser or a series of suction nozzles, for example.
e) Removing the pupae from the conveyance system.

Identification of the groups of pupae can be included in order to divide them by a watershed algorithm when sorting the pupae by size.

Likewise, the method can comprise a second scanning of the pupae by means of a positioning camera before elimination. This second scanning confirms the position and size of each pupa.

Preferably, the method will include a prior calibration process before operating and it can be done every time it is deemed necessary. This process starts by incorporating a blank element that can be marked by the eliminator, making three or more marks on the blank element with the conveyance system still or stopped, and detecting the marks by means of a camera and the artificial vision system. The change in coordinates or homography matrix between the two systems can therefore be reviewed.

When the conveyance system is a rotary conveyance system, the calibration can further comprise calculating the coordinates of the center of rotation thereof with respect to the eliminator. To that end, a second step is performed for incorporating a second blank element, making three or more marks by means of the eliminator with the conveyance system activated (on what corresponds to the same coordinates for the eliminator) and detecting the marks by means of a camera and the artificial vision system. These three or more points will define a circumference, the center of rotation of which is the desired one.

### DESCRIPTION OF THE DRAWINGS

The following drawings are included in order to better understand the invention.
Figure 1 shows a schematic depiction of an embodiment.
Figure 2 shows a histogram of the size of the pupae in an example of application for mosquitoes.

### DETAILED DISCLOSURE OF THE INVENTION

An embodiment of the invention will be briefly described below as an illustrative and non-limiting example thereof.

The invention comprises a device with a feeder (1) for feeding pupae (2) in their natural aquatic environment to a conveyance system (3), such as a rotary table, a conveyor belt, etc., provided that they meet the requirements that will be indicated below. The conveyance system (3) will preferably move at a constant and uniform (angular or linear) speed, which allows calculating the trajectory of each pupa (2). Speed will be controlled by the control system (4) formed by a programmable logic unit, a computer or any suitable system and will be synchronized with the rest of the device. The forward movement of the conveyance system (3) will preferably be controlled by servomotors and servocontrollers.

The conveyance system (3) will have a high-density metal mesh (5) on a translucent base (6) made of methacrylate, for example. The base (6) can be a mobile or fixed base. Both the mesh (5) and the base (6) allow the passage of light so as to not interfere with the process of recognizing the pupae (2) by means of artificial vision. Furthermore, the mesh (5) offers the strength required to withstand the firing of a laser (7), which is the preferred eliminator, without being destroyed. Furthermore, the mesh (5) allows removing the water from the feed, leaving the pupae (2) in dry form on the conveyance system (3). In the case of mosquito pupae (2), an example of mesh (5) has a 0.06 mm stainless steel wire and a 0.110 mm opening.

A lighting system (8), which can be LEDs, is arranged below the base (6). The light goes through the base (6) and the mesh (5), being filtered by both, through the pupae (2) and reaches one or more cameras (9, 9') located above the conveyance system (3). The cameras (9, 9') thereby obtain a high-resolution image of the mesh (5) and the pupae (2) under backlight, and image processing and recognition is made easier. It is possible, through less preferred, to use another arrangement for lighting and capturing images, but it makes recognition difficult. In these cases, the base (6) may not be translucent.

The preferred lighting system (8) is high-power (50 W) DC LEDs to prevent or reduce oscillations in the light intensity which make capturing by the cameras (9) and particularly software processing difficult. In contrast, an AC power supply may present oscillations at the grid frequency, generating recognition uncertainty.

The control system (4) recognizes the pupae (2), using their size difference to indicate which of the pupae must be eliminated generally by firing at them using the laser (7) which will preferably have a redirecting galvanometer (10) as it is the fastest and most effective method. The way to perform the selection will be described below. Once the pupae are subjected to the action of the laser (7), they are removed from the conveyance system (3) by means of pulses of water, air or another fluid so that they can be returned to the starting aquatic environment or to a suitable container.

The cameras (9, 9') can be divided according to their function into a sampling camera (9) and a positioning camera (9'). The images of the sampling camera (9) are used for measuring the size of the pupae (2) to calculate size distribution in a sampling area (SA), and the positioning camera (9') is used for calculating the position of the centroids, particularly of those pupae indicated for elimination in the firing area (FA) where the eliminator will act. However, both functions can be performed by a single camera (9, 9') either by overlapping both areas (SA, FA) or by calculating the position within the firing area (FA) based on the information obtained in the sampling area (SA).

Each element of the device has its own features: power supply, etc. Likewise, the eliminator (suction device, laser (7) and galvanometer (10)) or each camera (9, 9') can actually correspond to more than one element.

### EQUIPMENT OPERATION

The operating method of the device will be described for a conveyance system (3) corresponding to a rotary table. However, any person skilled in the art would know how to adapt this method to an alternative conveyance system (3).

With the rotary table (base (6) and mesh (5)) rotating at a constant, fully regulated angular speed, the feeder (1) gradually allows the pupae (2) to fall onto the mesh (5) in a homogenous manner, preventing the formation of groups of pupae (2) as much as possible. A group of pupae (2) can also be recognized by means of the artificial vision but with more errors, so it is preferable to avoid them. For example, the outlet of the feeder (1) can be formed by several small nozzles, such that the outflow is distributed over the entire useful surface. No clusters are produced when combining the foregoing with the movement of the conveyance system (3).

If there are two cameras (9, 9') in two different positions, the pupae (2) on the mesh (5) will first pass below the sampling camera (9) segmenting and parametrizing them for the first time (sampling area, SA). The pupae (2) then enter the firing area (FA) of the eliminator (generally the laser (7)). If the position of each pupa is to be known at this time, since the distance between both areas (SA, FA) as well as the speed are known, the control system (4) knows the position of each pupa (2) located in the sampling area (SA) when performing elimination with less error. This distance or time lag serves as a buffer for achieving the statistical significance required and allowing elimination in the firing area (FA) by sex.

The threshold is automatically determined based on this earlier information which allows identifying two groups of different populations corresponding respectively with males and females in a statistical significant manner. The individuals of the samples can be sorted by means of clustering techniques and using the k-mean algorithm or the like, for example.

In the case of using a laser (7), in order to prevent any position error, there will be arranged next to the laser (7) a positioning camera (9') displaying the firing area (FA) which is the same area covered by the galvanometer (10). The object of this positioning camera (9') is to recalculate the size of each individual in order to compare it to the threshold previously calculated in the preceding process and therefore determine if the pupa is male or female, and ascertain the coordinates. However, this information may have been retained from the sampling area (SA).

Through the positioning camera (9') the centroid of each pupa (2) can be ascertained by means of image analysis and the coordinates can be determined with respect to the coordinate system used by the galvanometer (10). The instantaneous firing sequences that the laser (7) must perform are determined with this information, sending the required information to the control module for controlling the position of the galvanometer (10) and activating the firing.

If there is only one camera (9, 9') and before firing with the laser (7) starts, a round will be performed based on which the histogram will be constructed, sorting and distributing the individuals by size. Based on that information the size threshold for differentiating between males and females will be calculated. That round can also be performed in a calibration process, such as the one that will be mentioned below. If the conveyance system (3) is a circular conveyance system, it involves going one round without extracting the pupae (2). In the case of linear conveyance systems (3), it may be required to introduce the discharged pupae (2) back into the feeder (1).

If there is only one camera (9, 9'), the firing area (FA) and the sampling area (SA) will preferably overlap, which makes the calibration that will be described below easier. In that case, object recognition for reviewing the threshold will focus on the inlet to the sampling area (FA) and the outlet of the sampling area (SA) can be used for verifying the position and sorting. Likewise, this verification can be omitted and the coordinates for elimination can be based on the correction of coordinates in a constant, precise and known movement of the conveyance system (3) and a clock inside the control system (4).

The recognition and artificial vision system for recognizing and viewing the pupae (2) is based on high-transfer rate, high-resolution (10 Mpx) cameras (9, 9') with a Gigabit output interface, a USB3 output interface or the like. Cameras (9, 9') of this type offer the required capture rate and precision in the image for segmenting the individuals. However, it requires a backlighting system. Cameras (9, 9') with other features may introduce more errors or force the conveyance system (3) to move more slowly.

With this lighting and cameras (9, 9') of this type, an image can be captured with an exposure time close to 2 ms. These extremely short exposure times allow eliminating distortions in the image due to movement of the conveyance system (3), giving rise to very sharp, high-resolution shots suitable for correct identification by size. At the same time it also allows achieving high image capture rates which in turn allow achieving industrial productivities in the male/female identification required by the process.

The artificial vision system is capable of segmenting the different individuals, isolating them from the background and determining the surface areas of each of them measured in number of square pixels. In addition to detecting the surface area, the software also identifies the position of the centroid of each pupa (2) which will subsequently be used for guiding the galvanometer (10) and for firing if the pupa has been sorted as having the target sex, for example, female.

Groups of pupae (2) that stick or come together forming islands sometimes form. In these cases, it is not possible to segment each individual directly and there is a need to resort to other ways, such as the use of the "watershed" algorithm which allows identifying the individuals of a group. This algorithm is more costly on a computational level; however, this problem has been handled by applying the algorithm only to the regions of the image containing the groups, i.e., the bodies the device recognizes as being too large to correspond to a single pupa (2). A good image segmentation rate per unit of time is therefore achieved as a whole.

Once the individuals of a frame of the sampling camera (9) have been segmented and their surface areas calculated, they become part of a dynamic data set (in the form of FIFO queue) used for sorting by sex.

To that end, this data set is subjected to a clustering technique based on the k-mean algorithm or another similar one. This algorithm is capable of automatically recognizing and sorting the two populations from among the data set and establishing a surface area size threshold, such that all the individuals below the threshold are considered males and the rest females. Given that there is always a certain degree of overlap between the tails of the bell curves associated with each population group, the software allows establishing a certain confidence interval which is parameterizable.

With this information, the software generates a dynamic database of individuals in the form of an FIFO (First Input First Output) queue listing a specific parameterizable number of the last acquired samples. Around 500 samples are considered in the tests. It is ultimately a sampling window.

With this information the device "learns" and determines a reference value or size threshold (in square pixels, for example, as it does not require conversion to real units) discriminating males from females. Preferably, the threshold is dynamic and is continuously updated, giving rise to a self-adaptive system. It is therefore independent of the different relative sizes that the different batches of pupae (2) may have and that depend on external factors such, as the amount of food obtained in the larval stage.

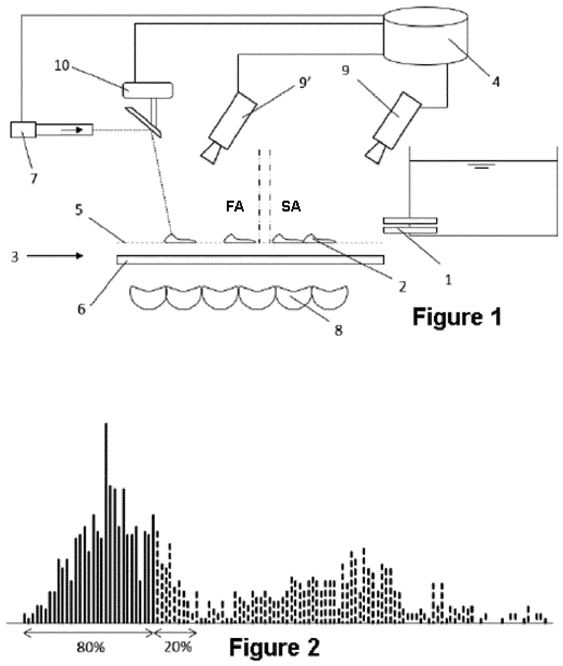

It is possible to verify the operation by printing a histogram representing the two populations and the established threshold point on the sample image, taking into account the established confidence interval. Figure 2 shows an example; the continuous lines correspond to those considered to be males and the dashed lines correspond to those considered to be females, in this case with an interval of 80% (i.e., 20% of the pupae (2) that would be considered to be males are selected as female). In this example, the number of males is significantly higher than the number of females.

Once the threshold is established, the positioning camera (9') adjacent to the galvanometer (10) again scans the pupae (2), ascertaining the surface areas and the positions of their centroids again. All the coordinates of the pupae (2) sorted as females (above the threshold) are added to a list representing the firing sequence, and this information is sent to the module which is in charge of controlling the position of the galvanometer (10).

### EQUIPMENT CALIBRATION

Before firing, the relationship between the coordinated systems of the positioning camera (9') and the galvanometer (10) must be established by means of a calibration process.

This calibration process can be extremely complex given the number of variables involved in correctly aligning the components which are part of the system: eliminator (suction device, laser (7) and galvanometer (10)), cameras (9, 9') and conveyance system (3), such as a rotary disk, for example. However, the invention comprises a way to automate system calibration, turning it into a very simple task that attains maximum precision.

The preferred calibration method is based on adjusting the components (when installing the system for the first time) as best as possible, but in an approximate manner. The fully automated calibration process consists of two steps:
- Calculating the homography matrix: This matrix establishes the relationship between the plane of the positioning camera (9') and the projection plane of the galvanometer (10). For the process, a blank element that can be marked by the eliminator is placed on the surface of the still conveyance system (3) and after starting the process, the eliminator acts three or more times (for example 4 times) on the blank element, giving rise to as many small marks as times the eliminator acts. The positioning camera (9') then detects the position of these marks and calculates the homography matrix linking the two systems. An example of a blank element for an eliminator operating by means of laser (7) would be a sheet of paper. For an eliminator in the form of a suction device, it would be a contrast powder providing contrast with respect to the base (6), the color difference with the base (6) (visible where the mark has been made by suctioning the contrast powder) can be detected by the camera (9, 9').
- Calculating the coordinates of the center of rotation of the mesh (5) in rotary conveyance systems (3) with respect to the coordinate system of the galvanometer (10). This step is necessary because the software must compensate for the respective positions of the pupae (2) due to the time periods that elapse from the time they are scanned until the time they are identified, their positions calculated and firing performed. To ascertain the center of rotation, it is again necessary to place a blank element on the mesh (5). In this case, the system will consecutively actuate the eliminator three or more times, while at the same time the mesh (5) rotates in a controlled manner. The generated points are recognized by the positioning camera (9') and the center of rotation with respect to the coordinate system of the galvanometer (10) is ascertained based on these points. This step is not necessary in the case of linear conveyance systems (3).

The system is completely calibrated and the parameters are stored in a configuration file after these two processes, which usually do not take more than two or three minutes. The system must not be recalibrated unless the orientation or position of one of the elements integrated therein is modified.

With the system duly calibrated, the software sends the sequence of coordinates corresponding to the centroids of the pupae (2) sorted for elimination (normally females) to the control module of the galvanometer (10), and the electronics are in charge of guiding the galvanometer (10) and activating the laser (7) autonomously.

A preferred example of laser (7) would be a 40 W CO₂ laser generator working in a wavelength that is well absorbed by organic material. With this power, it has been verified that pulses of 8 ms are sufficient for eliminating a pupa (2) with an initial setup time of another 8 ms for starting the sequence. These times could be reduced by using more powerful laser modules. By using a CO₂ laser (7), the galvanometer (10) requires an F-Theta type lens compatible with said laser (7). These lenses have the characteristic of converging the laser beam on one and the same plane regardless of the configuration of the angles of the mirrors making up the galvanometer (10).

## Claims

1. A device for selectively eliminating aquatic pupae of insects having sexual dimorphism, **characterized in that** it comprises:
a feeder (1) for feeding pupae (2) in an aquatic environment on;
a conveyance system (3) formed by a movable mesh (5) moving at a constant and uniform speed, and a base (6);
a lighting system (8);
one or more cameras (9, 9') connected to an artificial vision system locating the pupae (2) and measuring their size;
an eliminator for eliminating pupae (2) from the directable flow;
a control system (4) selecting pupae (2) having a size larger or smaller than a threshold for being eliminated;
and a removal system for removing the pupae (2) from the conveyance system (3).

2. The device according to claim 1, wherein the eliminator is a laser (7).

3. The device according to claim 2, wherein the laser (7) has a redirecting galvanometer (10).

4. The device according to claim 1 having a sampling camera (9) in a sampling area before a positioning camera (9') the field of vision of which coincides with the laser firing area (7).

5. The device according to claim 1, wherein the lighting system (8) is a backlighting system preferably by means of DC LEDs.

6. The device according to claim 1, wherein the conveyance system (3) is a rotary conveyance system.

7. The device according to claim 2, wherein the laser (7) is a CO₂ laser.

8. The device according to claim 1, wherein the eliminator is one or more suction devices.

9. A method for selectively eliminating aquatic pupae of insects having sexual dimorphism by means of the device according to claim 1, **characterized in that** it comprises the steps of:
a) feeding the pupae (2) in an aquatic environment to a conveyance system (3) with a movable mesh (5)
b) taking an image of all the pupae by means of at least one camera (9, 9');
c) sorting the size of the pupae (2) by means of artificial vision and defining a separation threshold between males and females;
d) eliminating selected pupae (2) above or below the threshold from the flow by means of an eliminator;
e) removing the pupae (2) from the conveyance system (3).

10. The method according to claim 9, wherein the threshold is calculated in a dynamic manner.

11. The method according to claim 9, wherein sorting the pupae (2) by size comprises identifying the groups and dividing them by a watershed algorithm.

12. The method according to claim 9 comprising a second scanning of the pupae (2) by means of a positioning camera (9') before elimination.

13. The method according to claim 9 comprising a prior calibration process by means of incorporating a blank element that can be marked by the eliminator, making three or more marks on the blank element by means of the eliminator with the conveyance system (3) stopped, and detecting the marks by means of a camera (9, 9') and the artificial vision system.

14. The method according to claim 13, wherein the conveyance system (3) is a rotary conveyance system and the calibration further comprises calculating the coordinates of the center of rotation thereof with respect to the eliminator by means of a second step of incorporating a second blank element, making three or more marks by means of the eliminator with the conveyance system (3) activated and detecting the marks by means of a camera (9, 9') and the artificial vision system.

15. The method according to claim 9, wherein the eliminator is a laser (7).

## Patentansprüche

1. Vorrichtung zur selektiven Beseitigung von aquatischen verpuppten Larven von Insekten, welche Geschlechtsdimorphismus aufweisen, **dadurch gekennzeichnet, dass** sie Folgendes umfasst:
ein Zuführungsmittel (1) zum Zuführen von verpuppten Larven (2) in einer aquatischen Umgebung auf;
einem Fördersystem (3) gebildet aus einem beweglichen Gitter (5), welches sich mit einer konstanten und gleichmäßigen Geschwindigkeit bewegt, und einer Grundfläche (6);
ein Beleuchtungssystem (8);
eine oder mehrere Kameras (9, 9') verbunden mit einem künstlichen Beobachtungssystem, welches die verpuppten Larven (2) lokalisiert und deren Größe misst;
ein Beseitigungsmittel zur Beseitigung der verpuppten Larven (2) aus dem ausrichtbaren Fluss;
ein Steuersystem (4), welches verpuppten Larven (2) mit einer größeren oder kleineren Größe als eine Schwelle auswählt, um sie zu beseitigen;
und ein Entnahmesystem zur Entnahme der verpuppten Larven (2) aus dem Fördersystem (3).

2. Vorrichtung nach Anspruch 1, wobei das Beseitigungsmittel ein Laser (7) ist.

3. Vorrichtung nach Anspruch 2, wobei der Laser (7) ein neuausrichtendes Galvanometer (10) aufweist.

4. Vorrichtung nach Anspruch 1, mit einer Abtastkamera (9) in einem Abtastbereich vor einer Positionierkamera (9') deren Sichtfeld mit dem Laserschussbereich (7) übereinstimmt.

5. Vorrichtung nach Anspruch 1, wobei das Beleuchtungssystem (8) ein Rückbeleuchtungssystem vorzugsweise mittels DC LEDs ist.

6. Vorrichtung nach Anspruch 1, wobei das Fördersystem (3) ein rotierendes Fördersystem ist.

7. Vorrichtung nach Anspruch 2, wobei der Laser (7) ein CO₂-Laser ist.

8. Vorrichtung nach Anspruch 1, wobei das Beseitigungsmittel einer oder mehrere Saugvorrichtungen ist.

9. Verfahren zur selektiven Beseitigung von aquatischen verpuppten Larven von Insekten, welche Geschlechtsdimorphismus aufweisen, mittels der Vorrichtung nach Anspruch 1, **dadurch gekennzeichnet, dass** es die folgenden Schritte umfasst:
a) das Zuführen der verpuppten Larven (2) in einer aquatischen Umgebung zu einem Fördersystem (3) mit einem beweglichen Gitter (5)
b) das Aufnehmen eines Bildes aller verpuppten Larven mittels mindestens einer Kamera (9, 9');
c) das Sortieren der Größe der verpuppten Larven (2) mittels künstlicher Sicht und das Definieren einer Trennschwelle zwischen Männchen und Weibchen;
d) das Beseitigen der ausgewählten verpuppten Larven (2) über oder unter der Schwelle aus dem Fluss mittels eines Beseitigungsmittels;
e) das Entnehmen der verpuppten Larven (2) aus dem Fördersystem (3).

10. Verfahren nach Anspruch 9, wobei die Schwelle dynamisch berechnet wird.

11. Verfahren nach Anspruch 9, wobei das Sortieren der verpuppten Larven (2) anhand der Größe das Identifizieren der Gruppe und das Unterteilen derselben mittels eines Watershed-Algorithmus umfasst.

12. Verfahren nach Anspruch 9, umfassend eine zweite Abtastung der verpuppten Larven (2) mittels einer Positionierkamera (9') vor der Beseitigung.

13. Verfahren nach Anspruch 9, umfassend einen vorherigen Kalibrierungsprozess mittels des Einbeziehens eines blanken Elements, welches mittels des Beseitigungsmittels markiert werden kann, sodass drei oder mehr Markierungen auf dem blanken Element mittels des Beseitigungsmittels mit gestopptem Fördersystem (3) hergestellt werden, und die Markierungen mittels einer Kamera (9, 9') und des künstlichen Beobachtungssystems detektiert werden.

14. Verfahren nach Anspruch 13, wobei das Fördersystem (3) ein rotierendes Fördersystem ist und die Kalibrierung zusätzlich das Berechnen der Koordinaten des Rotationszentrums desselben in Bezug auf das Beseitigungsmittel mittels eines zweiten Schrittes des Einbeziehens eines zweiten blanken Elements, sodass drei oder mehr Markierungen mittels des Beseitigungsmittels mit aktiviertem Fördersystem (3) hergestellt werden und die Markierungen mittels einer Kamera (9, 9') und des künstlichen Beobachtungssystems detektiert werden, umfasst.

15. Verfahren nach Anspruch 9, wobei das Beseitigungsmittel ein Laser (7) ist.

## Revendications

1. Dispositif pour l'élimination sélective des chrysalides aquatiques d'insectes qui présentent un dimorphisme sexuel, **caractérisé en ce qu'**il comprend :
un dispositif d'alimentation (1) pour alimenter des chrysalides (2) dans un milieu aquatique sur ;
un système de transport (3) constitué d'un filet mobile (5) qui se déplace à vitesse constante et uniforme, et une base (6) ;
un système d'éclairage (8) ;
une ou plusieurs caméras (9, 9') reliées à un système de vision artificielle qui localise les chrysalides (2) et mesure leur taille ;
un éliminateur pour éliminer les chrysalides (2) à partir de l'écoulement orientable ;
un système de contrôle (4) qui sélectionne les chrysalides (2) ayant une taille supérieure ou inférieure à un seuil pour être éliminées ;
et un système de retrait pour retirer les chrysalides (2) à partir du système de transport (3).

2. Dispositif selon la revendication 1, dans lequel l'éliminateur est un laser (7).

3. Dispositif selon la revendication 2, dans lequel le laser (7) possède un galvanomètre de réorientation (10).

4. Dispositif selon la revendication 1, qui possède une caméra d'échantillonnage (9) dans une zone d'échantillonnage avant une caméra de positionnement (9') dont le champ de vision coïncide avec la zone de tir par laser (7).

5. Dispositif selon la revendication 1, dans lequel le système d'éclairage (8) est un système de rétroéclairage de préférence par le biais de LED de CC.

6. Dispositif selon la revendication 1, dans lequel le système de transport (3) est un système de transport rotatif.

7. Dispositif selon la revendication 2, dans lequel le laser (7) est un laser de CO₂.

8. Dispositif selon la revendication 1, dans lequel l'éliminateur est un ou plusieurs dispositifs d'aspiration.

9. Procédé pour l'élimination sélective des chrysalides aquatiques d'insectes qui présentent un dimorphisme sexuel par le biais du dispositif selon la revendication 1, **caractérisé en ce qu'**il comprend les étapes consistant à :
a) alimenter les chrysalides (2) dans un milieu aquatique à un système de transport (3) avec un filet mobile (5) ;
b) prendre une image de toutes les chrysalides par le biais d'au moins une caméra (9, 9') ;
c) classer la taille des chrysalides (2) par le biais de vision artificielle et définir un seuil de séparation entre mâles et femelles ;
d) éliminer des chrysalides (2) sélectionnées au-dessus ou au-dessous du seuil à partir de l'écoulement par le biais d'un éliminateur ;
e) retirer les chrysalides (2) du système de transport (3).

10. Procédé selon la revendication 9, dans lequel le seuil est calculé d'une manière dynamique.

11. Procédé selon la revendication 9, dans lequel la classification des chrysalides (2) par taille comprend l'identification des groupes et leur division par un algorithme de ligne de partage des eaux.

12. Procédé selon la revendication 9, comprenant un second scannage des chrysalides (2) par le biais d'une caméra de positionnement (9') avant l'élimination.

13. Procédé selon la revendication 9, comprenant une procédure de calibration préalable par le biais de l'incorporation d'un élément en blanc pouvant être marqué par l'éliminateur, la réalisation de trois marques ou plus sur l'élément en blanc par le biais de l'éliminateur avec le système de transport (3) arrêté, et la détection des marques par le biais d'une caméra (9, 9') et le système de vision artificielle.

14. Procédé selon la revendication 13, dans lequel le système de transport (3) est un système de transport rotatif et la calibration comprend en outre le calcul des coordonnées de son centre de rotation par rapport à l'éliminateur par le biais d'une seconde étape d'incorporation d'un second élément en blanc, la réalisation de trois marques ou plus par le biais de l'éliminateur avec le système de transport (3) activé et la détection des marques par le biais d'une caméra (9, 9') et le système de vision artificielle.

15. Procédé selon la revendication 9, dans lequel l'éliminateur est un laser (7).
